# EUROPEAN PATENT SPECIFICATION

(11) **EP 2 308 383 B1**
(45) Date of publication and mention of the grant of the patent: **29.06.2016**
(21) Application number: 10251776.0
(22) Date of filing: 08.10.2010
(51) Int. Cl.: A61B 17/02, A61B 17/04

(54) **Internal retractor systems**
Interne Retraktorsysteme
Systèmes rétracteurs internes

(30) Priority: 09.10.2009 US 250072 P; 09.10.2009 US 250074 P; 04.10.2010 US 896941
(43) Date of publication of application: 13.04.2011
(73) Proprietor: Covidien LP, Mansfield, MA 02048 (US)
(72) Inventor: Rodrigues, Anibal, Jnr., Milford, CT 06460 (US); Ebner, Timothy D., New Haven, CT 06511 (US)
(74) Representative: Soames, Candida Jane

(56) References cited:
- WO-A1-03/096907
- WO-A1-2010/099327
- WO-A2-2007/149555
- WO-A2-2009/017680
- US-A1- 2008 071 306
- US-A1- 2009 062 618

## Description

### CROSS REFERENCE TO RELATED APPLICATIONS

The present application claims priority to United States Provisional Application Serial No. 61/250,072, filed October 9, 2009, entitled "MESH RETRACTORS WITH ADJUSTERS" and United States Provisional Application Serial No. 61/250,074, filed October 9, 2009, entitled "INTERNAL TISSUE ANCHORS".

### BACKGROUND

### 1. Technical Field

The present disclosure relates to systems and devices for retracting organ and/or body tissue during surgical procedures and, more particularly, to surgical mesh or slings for retracting or positioning body tissue and/or body organs during minimally invasive surgery.

### 2. Background of the Related Art

As a result of the recent technological improvements in surgical instruments, surgical procedures, using minimally invasive techniques (e.g., endoscopic, laparoscopic, etc.), are routinely performed that cause less trauma to the patient.

In endoscopic and laparoscopic surgical procedures, it is often necessary to provide instrumentation to move or manipulate tissue and/or organs located in the area of operation. Generally, laparoscopic surgical procedures involve the introduction of a gas, such as, carbon dioxide, to insufflate a body cavity, e.g., the abdomen, to provide a working area for the surgeon. A trocar device is utilized to puncture the peritoneum to provide an access port by way of a cannula through the abdominal wall for the introduction of surgical instrumentation. After puncturing the peritoneum, the abdomen is insufflated. Generally, a trocar/cannula is placed through the abdominal wall for each piece of surgical instrumentation which is necessary to carry out the surgical procedure. In this manner, the surgeon may view the surgical site through an endoscope provided through a first trocar/cannula, and utilize a second trocar/cannula to introduce a surgical instrument such as a grasper, scissor, clip applier, stapler and any other surgical instrument which may be necessary during the particular surgical procedure.

Although the insufflation gas expands the abdomen to permit the surgeon to view the surgical site, it is often necessary to manipulate the internal organ or tissues to provide a clear path to the surgical objective. In the past, grasping tools have been utilized which pull on the organ or tissues to move them out of the way to provide a clear visual path for the surgeon. Endoscopic retractor mechanisms also have been developed which are utilized to push and hold the tissue or organ away from the surgical site.

Such grasping tools and retractor mechanisms have a disadvantage in that the surgeon operating the tools is required to use one hand to operate the grasping tool or retractor mechanism while using their other hand to perform the surgical procedure. Accordingly, a need exists for an internal tissue retractor that retracts and maintains tissue and/or organs in a retracted position while allowing a surgeon to use both hands during a surgical procedure.

Surgeons may employ the use of surgical mesh or slings to retract tissue and/or organs while performing a procedure. The use of such meshes or slings is limited in that they can not be easily manipulated to change the position of the tissue and/or organs that are being retracted.

US 2008/ 071306 describes a retractor system having an anchor, anchoring component, cam cleat, suture and attachment device, the suture operable to suspend tissue from an abdominal wall.

### SUMMARY

A retractor system in accordance with Claim 1 is provided.

The retractor system provided includes at least one anchor having a base, an anchoring component attached to one end of the base and a cam cleat attached to another end of the base. The system also includes a suture having an attachment end, and an attachment device coupled to the attachment end of the suture, the attachment device being configured to attach to tissue and/or organs. The suture is operable to suspend tissue and/or organs from an abdominal wall at a desired length by pulling the suture through the cam cleat. The suture has a hard coated leader facilitating the suture to be passed through the cam cleat.

Various embodiments of the invention are found in the dependent claims.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 depicts a retractor system in accordance with the present disclosure;
Fig. 2A is a side view of a retractor system in accordance with the present disclosure;
Fig. 2B is a top view of the sling of Fig. 2A;
Fig. 3A depicts a retractor system according to the present disclosure;
Fig. 3B depicts a retractor system according to the present disclosure;
Fig. 4A depicts a abdominal wall anchor according to an embodiment of the present disclosure;
Fig. 4B depicts a cam cleat according to an embodiment of the present disclosure;
Figs. 5A through 5I depict abdominal wall anchors according to embodiments of the present disclosure;
Figs. 6A through 6F depict tissue attachment devices and
Figs. 7A and 7B depicts internal tissue anchors

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the abdominal wall and the term "distal" refers to the end of the apparatus which is farther away from the abdominal wall. The term "clinician" refers to any medical professional (i.e., doctor, surgeon, nurse, or the like) performing a medical procedure involving the use of embodiments described herein.

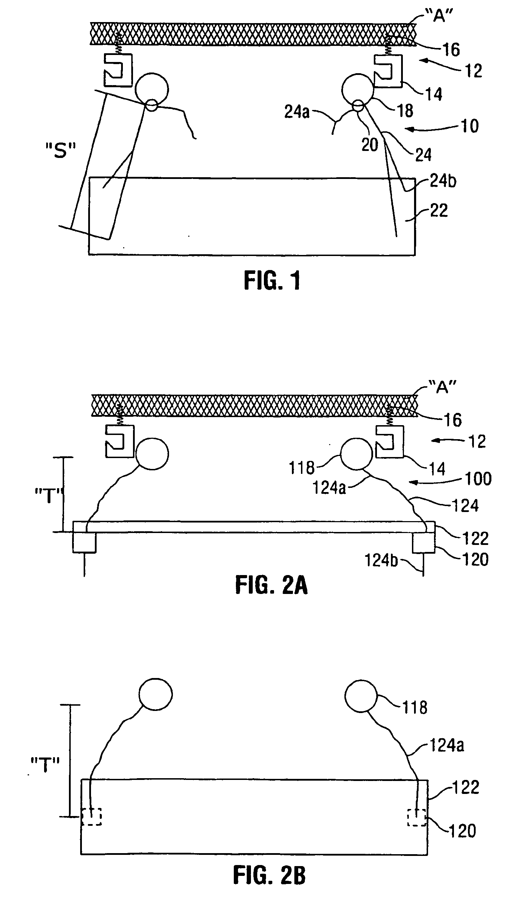

Fig. 1 depicts a retractor system 10 of the present disclosure. As shown in Fig. 1, anchors 12 are coupled to soft tissue, e.g., abdominal wall "A". Each anchor 12 has a hook shaped distal end 14 and may be formed from an easily absorbable material. Hook shaped distal end 14 is coupled to a helical coil 16, such as a titanium helical coil, which is coupled to abdominal wall "A".

Although Fig. 1 depicts an anchor 12 with a hook shaped distal end 14 and a helical coil 16, other types of anchors may be used with embodiments of the present disclosure. Other anchors that may be used include, but are not limited to, magnetic anchors, anchors having a wire loop, anchors having a sling coupled thereto. Additionally, other methods may be employed to couple the anchor to abdominal wall "A", such as sutures, staples, an I-clip, spikes, tacks or the like.

A ring 18 is suspended from each hook shaped distal end 14. Attached to each ring 18 is an adjuster 20 that is used to manipulate the surgical mesh or sling 22 as will be described below. Sling 22 can be made from any biocompatible material suitable for use inside a body cavity. Sling 22 is suspended from ring 18 using a wire or suture 24 that extends trough adjuster 20. Distal end 24b of suture 24 is coupled to sling 22 while proximal end 24a of suture 24 is threaded trough adjuster 20. Although Fig. 1 depicts suture 24 attached to the corners of sling 22, suture 24 can be attached anywhere on sling 22. Further, a single adjuster 20 may be utilized or multiple adjusters may be utilized.

During a surgical procedure, after a trocar pierces the peritoneum and prior to insufflating the abdomen, anchors 12 are coupled to abdominal wall "A" by one of the methods described above. A clinician places sling 22 under tissue and/or organs and then suspend the tissue and/or organs by suspending ring 18 from hook shaped distal end 14. The clinician can then adjust the suspension height or length "S" by pulling on proximal end 24a or distal end 24b. For instance, if the clinician desires to reduce length "S", then the clinician would pull on the proximal end 24a of suture 24 while holding adjuster 20 using clips or a grasping tool. If the clinician desires to increase length "S", then the clinician would pull on the distal end 24b of suture 24 while holding adjuster 20 using clips or a grasping tool.

Turning to Figs. 2A and 2B, a retractor system of the present disclosure is shown generally as 100. Retractor system 100 includes an anchor 12 having a hook shaped distal end 14 and helical coil 16 coupled to an abdominal wall "A". As shown in Figs. 2A and 2B, sling 122 has a suture 124 having a proximal end 124a connected to ring 118. Ring 118 may be replaced with a hook, suture or a needle that could be coupled directly to the abdominal wall without an anchor. Distal end 124b of suture 124 extends through sling 122 through an adjuster 120.

When a clinician uses the system described above with regards to Figs. 2A and 2B, the clinician places tissue and/or organs in sling 122. After which, the clinician, attaches distal end 124b to adjuster 120 through sling 122 and attaches proximal end 124a to anchor 12. When sling 122 is used to retract tissue and/or organs, the clinician can adjust the length "T" of suture 124 by moving the adjuster 120 toward the proximal end 124a or distal end 124b. For instance, if the clinician desires to reduce length "T", then the clinician would pull adjuster 120 toward proximal end 124a while holding distal end 124b of suture 124 using clips or a grasping tool. If the clinician desires to increase length "T", then the clinician would pull adjuster 120 toward distal end 124b while holding distal end 124b of suture 124 using clips or a grasping tool.

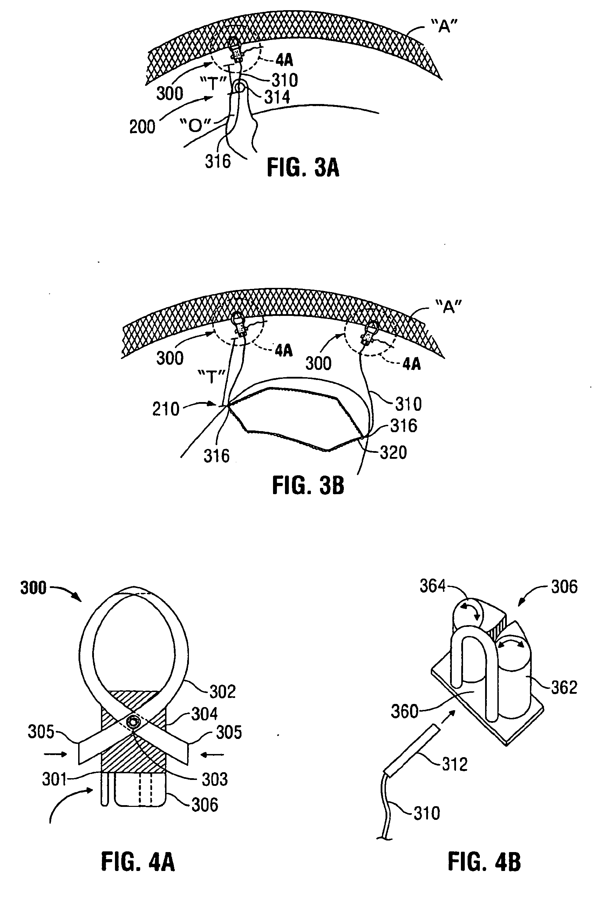

Turning to Figs. 3A and 3B, internal retractor systems of the present disclosure are shown generally as 200 and 210 respectively. Internal retractor system 200 of Fig. 3A includes an abdominal wall anchor 300, a suture 310 and a pledget 314. Anchor 300, which will be described in more detail hereinbelow with regard to Figs. 4A and 4B, is attached to abdominal wall "A". Suture 310 is connected to anchor 300 in a manner to be described hereinbelow such that the length "T" of suture 310 may be adjusted thereby adjusting the suspension height of tissue or organ "O". As shown in Fig. 3A, a pledget 314 is attached at one end of suture 310. Pledget 314 is used to suspend organ "O" from abdominal wall "A". Pledget 314 may include an adhesive, barbs, or any other means that can be used to attach the organ "O" to pledget 314. Alternatively, and as shown in Fig. 3B, internal retractor system 210 may include multiple anchors 300 with each anchor 300 being connected to a one end of a sling 320 used to retract tissue or organs.

Turning now to Figs. 4A and 4B, Fig. 4A depicts an abdominal wall anchor 300 and Fig. 4B depicts an adjuster or cam cleat 306 used with anchor 300. Anchor 300 includes a base 304 having a cam cleat 306 attached to end 301 of base 304. Base 304 may also include an anchoring component such as towel clamp pincers 302 on a torsion spring 304. Ends 305 are squeezed or compressed together causing pincers 302 to open. When ends 305 are released, pincers 302 bite into the abdominal wall as shown in Figs. 3A and 3B.

Fig. 4B depicts a cam cleat 306 that is attached to base 304. Cam cleat 306 has two spring loaded cams 362 and 364 that pinch suture 310 allowing the length of suture 310 to be adjusted easily and quickly released under load. Leader 312 of suture 310 is coated to make leader 312 hard which makes it easier to use suture 310 with cam cleat 306.

During an endoscopic or laparoscopic procedure, anchor 300 is attached to the abdominal wall "A". End 316 of suture 310 is attached to tissue or organ "O" using a pledget 314, sling 320 or any other means described herein. Leader 312 is then fed through an aperture 360 in cam cleat 306 between the two spring loaded cams 362 and 364. Thereafter, a clinician can easily adjust the length "T" of suture 310 by pulling on leader 312 through cam cleat 306 or applying a load to cam cleat 306 to release suture 310.

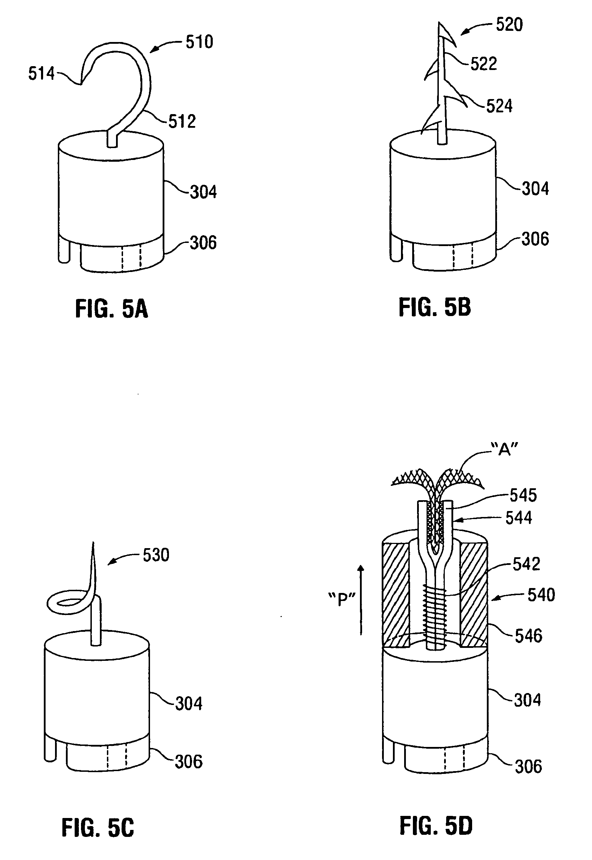

Figs. 5A through 5I depict different anchoring components that may be used to attach base 304 and cam cleat 306 to an abdominal wall. As shown in Fig. 5A, an anchor 510 includes a hook 512 with a sharp end 514. Sharp end 514 may be used to puncture the abdominal wall to secure anchor 510 to the abdominal wall. Fig. 5B depicts an anchor 520 that includes a spike 522 having a number of barbs 524 that may be used to secure anchor 520 to the abdominal wall. Fig. 5C depicts an anchor 530 that may be a helical coil such as a titanium helical coil that is coupled to abdominal wall.

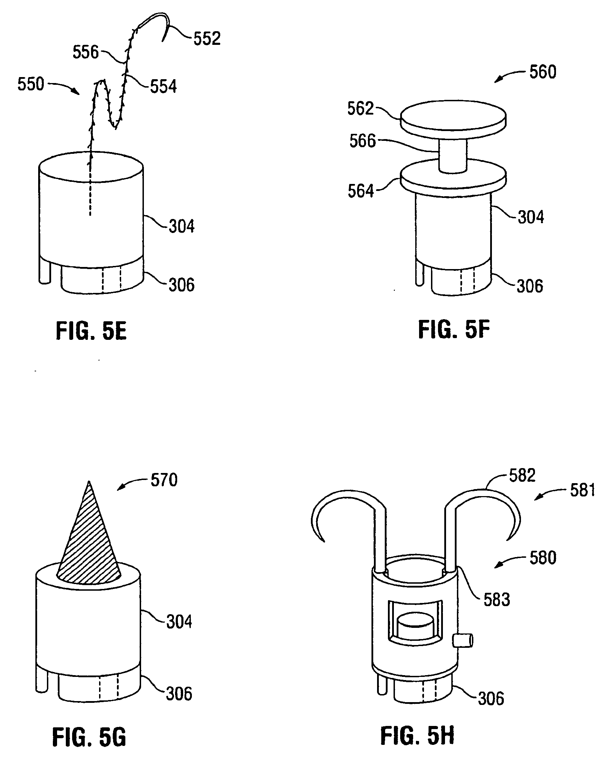

Fig. 5D depicts an anchor 540 having a tube 546 with spring 542 and clamp 544 therein. Clamp 544 grasps abdominal wall "A" and then tube 546 is moved in proximal direction "P" thereby compressing the jaws 545 of clamp 544 which pinches the abdominal wall "A" and secures anchor 540 thereto. Fig. 5E depicts an anchor 550 that uses a suture 554 with a needle 552 attached thereto. Needle 552 is passed through the abdominal wall to secure anchor 550 to the abdominal wall. Suture 554 may include a number of barbs 556.

Fig. 5F depicts an I-clip 560 attached to base 304. I-clip 560 is placed through an aperture in the abdominal wall such that the abdominal wall rests in between flanges 562 and 564. A tube or lumen 566 connects flange 562 to flange 564. Flanges 562 and 564 and lumen 566 may be formed from a single piece or may be removably coupled to each other. Fig. 5G depicts a tack 570, which pierces an abdominal wall and is secured therein by twisting tack 570 in a circular motion.

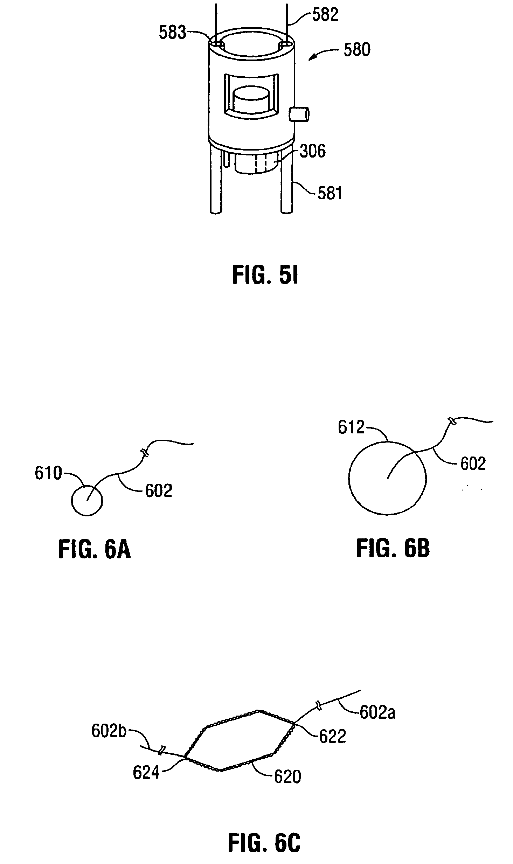

Figs. 5H and 5I depict another anchoring component in accordance with an embodiment of the present disclosure. An anchor 580 is provided that can be attached to abdominal wall "A". Anchor 580 has least two wire hooks 581 made from Nitinol or any other shape memory alloy. Shape memory alloys undergo large deformation under stress, yet regain their intended shape once the metal is unloaded again. As shown in Fig. 5H, hook 581 has a proximal end 582 used to couple the anchor 580 to abdominal wall "A". When a surgeon pulls hook 581 down, proximal end 582 is straightened and retracted into anchor 580 in chamber 583 (Fig. 5I). If the anchor is coupled to the abdominal wall "A", pulling down on the hook 581 releases the anchor 580 from the abdominal wall "A". Chamber 583 applies a force to proximal end 582 keeping it straight. When hook 581 is pushed upward such that chamber 583 no longer applies a force to end 582, hook 581 regains its intended shape as shown in Fig. 5H. This drives the hook 581 into abdominal wall "A" thereby securing the anchor therein. A cam cleat 306, as described above with regard to Fig. 4B, is coupled to the distal end of anchor 580. Hook(s) 581 may be actuated by a lever, spring, button, switch or the like (not shown).

Figs. 6A through 6F depict different tissue attachment devices for attaching tissue and/or organs to the abdominal wall anchors described hereinabove. Fig. 6A depicts a suture 602 having a pledget 610 (e.g., a 1 cm diameter pledget) attached thereto. Pledget 610 may include an adhesive, barbs or any other means that may be used to attach pledget 610 to tissue and/or organs. Fig. 6B depicts a suture 602 attached to a bigger pledget 612 (e.g., a 5 cm diameter pledget).

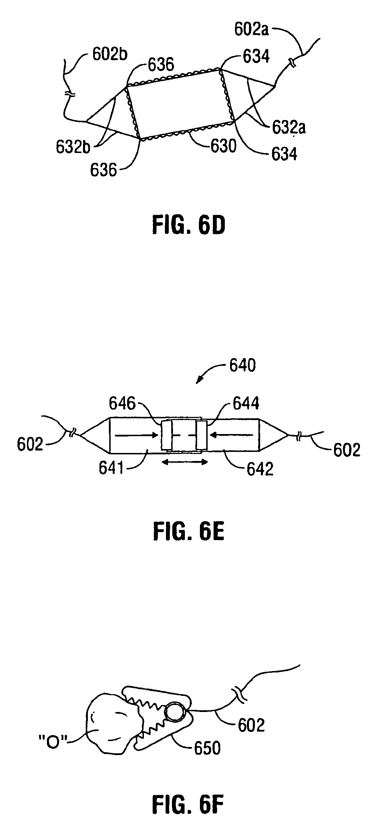

Figs. 6C through 6E depict different slings that may be used to retract tissue and or organs. As shown in Fig. 6C, suture 602a is connected to end 622 of sling 620 while suture 602b is connected to end 624 of sling 620. Alternatively, each suture lead may be connected to multiple points on a sling as shown in Fig. 6D. As shown in Fig. 6D, suture 602a branches off into leads 632a which are coupled to corners 634 of sling 630 while suture 602b branches off into leads 632b which are coupled to corners 636 of sling 630. Fig. 6E depicts an adjustable sling that may be used to retract tissue and/or organs. The length of sling 640 may be adjusted by moving sling 641 relative to sling 642. Sling 641 and sling 642 each have adjustment members 646 and 644, respectively, that can be used to shorten the length of sling 640 or increase the length of sling 640. Slings 620, 630 and 640 are placed under tissue and/or organs and then sutures 602a and 602b are attached to one of the abdominal wall anchors described above.

Fig. 6F depicts an alligator clamp 650 at one end of suture 602. Alligator clamp 650 clamps on to tissue or organ "O" to suspend the tissue and/or organ "O" from the abdominal wall.

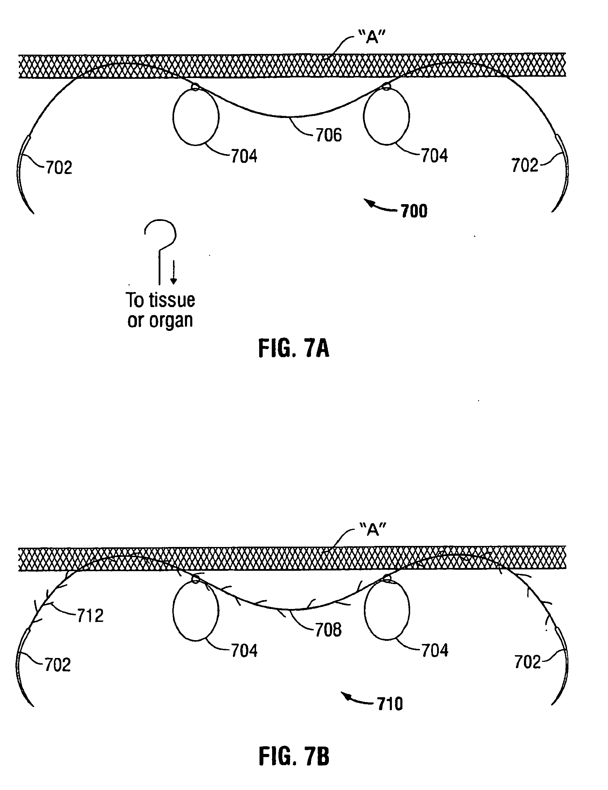

Figs. 7A and 7B depict internal tissue anchor systems 700 and 710, respectively. System 700 includes a suture 706 that can be threaded through abdominal wall "A" using one of needles 702. Suture 706 may include at least one anchoring ring or loop 704 from which tissue or organs may be suspended using a hook or any other method described hereinabove. Anchoring ring 704 may be coupled to suture 706 by any conventional means. Although Fig. 7A depicts a system 700 that includes two needles 702 and two anchoring rings 704, any number of needles 702 and corresponding anchoring rings 704 may be used. For instance, suture 706 may include a single thread having a single needle 702 with a single anchoring ring 704 or a suture 706 having multiple threads emanating from a single point where each thread has a corresponding needle 702 and anchoring ring 704 may be used. Suture 706 and anchoring rings 704 may be composed of standard suture material or an absorbable material that can be left inside the abdominal cavity thereby eliminating the worry of a non-absorbable device being lost or accidentally left in the abdominal cavity. Anchoring rings 704 may be disposed on suture 706 before suture 706 is disposed in the abdominal cavity or they may be added onto suture 706 after suture 706 is threaded into the abdominal wall "A".

Alternatively, suture 706 may incorporate V-Loc technology as shown in Fig. 7B (suture 708). For instance, as shown in Fig. 7B, suture 708 includes unidirectional shallow barbs 712 that may have a circumferential distribution (not shown). Barbs 712 are evenly spaced throughout suture 708 to grasp the abdominal wall "A" at numerous points thereby spreading tension across the abdominal wall "A" and reducing trauma to the abdominal wall "A".

The retractor system may use a proximal adjuster that is coupled to an anchor (e.g., as shown in Fig. 1) and a distal adjuster below a sling (e.g., as shown in Fig. 2A). By using a retractor system having a proximal adjuster and a distal adjuster, the clinician may change the suspension length of the tissue and/or organs by using either or both of the adjusters based upon the clinician's access to and ease of adjusting either adjuster at any point during the surgical procedure.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the disclosure.

## Claims

1. A retractor system comprising:
at least one anchor (300) having a base (304) an anchoring component attached to one end of the base and a cam cleat (306) attached to another end of the base;
a suture (310) comprising an attachment end (316),
an attachment device (22, 314, 316) coupled to the attachment end of the suture, the attachment device being configured to attach to or to support tissue and/or organs,
the suture being operable to suspend the tissue and/or organs from an abdominal wall at a desired length by pulling the suture through the cam cleat **characterised in that**
the suture has a hard coated leader (312) facilitating the suture to be passed through the cam cleat.

2. The retractor system according to claim 19, wherein the attachment device is surgical mesh.

3. The retractor system according to claim 1, wherein the anchoring component includes towel pincers on a torsion spring.

4. The retractor system according to claim 1, wherein the cam cleat -comprises two spring loaded cams.

5. The retractor system according to claim 1, wherein the anchor includes a hook with a sharp end.

6. The retractor system according to claim 1 wherein the anchor includes a spike having barbs.

7. The retractor system according to claim 1, wherein the anchor is a helical coil.

8. The retractor system according to claim 7, wherein the helical coil is made from titanium.

9. The retractor system according to claim 1, wherein the anchor has a tube with a spring and clamp therein.

10. The retractor system according to claim 1, wherein the anchor uses a suture with a needle attached thereto.

11. The retractor system according to claim 3, wherein the anchor is an I-clip.

12. The retractor system according to claim 1, wherein the anchor is a tack.

13. The retractor system according to claim 1, wherein the anchor has two wire shape memory alloy hooks

## Patentansprüche

1. Ein Retraktorsystem aufweisend:
wenigstens einen Anker (300) mit einer Basis (304), einer an einem Ende von der Basis befestigten Verankerungskomponente und einer an einem anderen Ende von der Basis befestigten Curry-Klemme (306);
ein Faden (310), der ein Befestigungsende (316) aufweist;
eine Befestigungsvorrichtung (22, 314, 316), die mit dem Befestigungsende von dem Faden verbunden ist, wobei die Befestigungsvorrichtung konfiguriert ist, um Gewebe und/oder Organe zu befestigen oder zu stützen,
wobei der Faden betriebsbereit ist, um das Gewebe und/oder Organe an einer abdominalen Wand mit einer gewünschten Länge aufzuhängen, indem der Faden durch die Curry-Klamme hindurch gezogen wird, **dadurch gekennzeichnet, dass**
der Faden einen hartbeschichteten Führer (312) hat, der es ermöglicht, dass der Faden durch die Curry-Klemme hindurchgeht.

2. Das Retraktorsystem nach Anspruch 1, wobei die Befestigungsvorrichtung ein chirurgisches Netz ist.

3. Das Retraktorsystem nach Anspruch 1, wobei die Verankerungskomponente Tuchzangen an einer Torsionsfeder umfasst.

4. Das Retraktorsystem nach Anspruch 1, wobei die Curry-Klemme zwei federbelastete Nocken aufweist.

5. Das Retraktorsystem nach Anspruch 1, wobei der Anker einen Haken mit einem scharfen Ende umfasst.

6. Das Retraktorsystem nach Anspruch 1, wobei der Anker einen Stachel mit Widerhaken umfasst.

7. Das Retraktorsystem nach Anspruch 1, wobei der Anker eine spiralförmige Spule ist.

8. Das Retraktorsystem nach Anspruch 7, wobei die spiralförmige Spule aus Titan hergestellt ist.

9. Das Retraktorsystem nach Anspruch 1, wobei der Anker ein Rohr mit einer Feder und Klammer darin hat.

10. Das Retraktorsystem nach Anspruch 1, wobei der Anker einen Faden mit einer daran befestigten Nadel verwendet.

11. Das Retraktorsystem nach Anspruch 1, wobei der Anker ein I-Clip ist.

12. Das Retraktorsystem nach Anspruch 1, wobei der Anker ein Reißnagel ist.

13. Das Retraktorsystem nach Anspruch 1, wobei der Anker zwei drahtförmige Gedächtnislegierungshaken hat.

## Revendications

1. Système rétracteur comprenant :
au moins une ancre (300) ayant une base (304), un composant d'ancrage attaché à une extrémité de la base et un taquet coinceur (306) attaché à une autre extrémité de la base ;
une suture (310) comprenant une extrémité d'attachement (316) ;
un dispositif d'attachement (22, 314, 316) couplé à l'extrémité d'attachement de la suture, le dispositif d'attachement étant configuré pour s'attacher à ou supporter du tissu et/ou des organes,
la suture pouvant être mise en oeuvre pour suspendre le tissu et/ou les organes provenant d'une paroi abdominale à une longueur souhaitée en tirant la suture à travers le taquet coinceur, **caractérisé en ce que**
la suture a une amorce à couche dure (312) facilitant le passage de la suture à travers le taquet coinceur.

2. Système rétracteur selon la revendication 1, dans lequel le dispositif d'attachement est un treillis chirurgical.

3. Système rétracteur selon la revendication 1, dans lequel le composant d'ancrage inclut des tenailles fixe-compresses sur un ressort de torsion.

4. Système rétracteur selon la revendication 1, dans lequel le taquet coinceur comprend deux cames chargées par ressort.

5. Système rétracteur selon la revendication 1, dans lequel l'ancre inclut un crochet avec une extrémité pointue.

6. Système rétracteur selon la revendication 1, dans lequel l'ancre inclut une pointe ayant des picots.

7. Système rétracteur selon la revendication 1, dans lequel l'ancre est une bobine hélicoïdale.

8. Système rétracteur selon la revendication 7, dans lequel la bobine hélicoïdale est faite de titane.

9. Système rétracteur selon la revendication 1, dans lequel l'ancre a un tube avec un ressort et une attache en son sein.

10. Système rétracteur selon la revendication 1, dans lequel l'ancre utilise une suture avec une aiguille attachée à celle-ci.

11. Système rétracteur selon la revendication 1, dans lequel l'ancre est une agrafe en I.

12. Système rétracteur selon la revendication 1, dans lequel l'ancre est un clou à tête.

13. Système rétracteur selon la revendication 1, dans lequel l'ancre a deux crochets filaires en alliage à mémoire de forme.
